# EUROPEAN PATENT APPLICATION

(11) **EP 3 753 511 A1**
(43) Date of publication of application: **23.12.2020**
(21) Application number: 20180350.9
(22) Date of filing: 16.06.2020
(51) Int. Cl.: A61B 17/34

(54) **SEALS FOR SURGICAL ACCESS ASSEMBLIES**

(30) Priority: 21.06.2019 US 201916448864
(71) Applicant: Covidien LP, Mansfield, MA 02048 (US)
(72) Inventor: PILLETERE, Roy, North Haven, CT Connecticut 06473 (US); LAPIERRE, Nicolette, Windsor Locks, CT Connecticut 06096 (US); DININO, Matthew, Newington, CT Connecticut 06111 (US); EBERSOLE, Garrett, Hamden, CT Connecticut 06517 (US); BARIL, Jacob, Norwalk, CT Connecticut 06851 (US); DESJARDIN, Kevin, Prospect, CT Connecticut 06712 (US); BROWN, Eric, Madison, CT Connecticut 06443 (US)
(74) Representative: Maschio, Antonio

(57) **Abstract**

A surgical access assembly includes an instrument valve housing and a valve assembly disposed within a cavity of the instrument valve housing. The valve assembly includes a guard assembly, a seal assembly disposed adjacent to the guard assembly, and a centering mechanism for maintaining the seal assembly and guard assembly centered within the cavity of the instrument valve. The guard assembly includes first and second guard members.

## Description

### FIELD

The present disclosure relates to surgical access assemblies for minimally invasive surgery. More particularly, the present disclosure relates to valve assemblies for surgical access assemblies.

### BACKGROUND

In order to facilitate minimally invasive surgery, a working space must be created in the desired surgical space. An insufflation fluid, typically CO2, is introduced into the abdomen of the patient to create an inflated state called a pneumoperitoneum. Surgical access assemblies are utilized to allow the introduction of surgical instrumentation and endoscopes (or other visualization tools). These surgical access assemblies maintain the pressure for the pneumoperitoneum, as they have one or more seals that adapt to the surgical instrumentation. Typically, a "zero-seal" in the surgical access assembly seals the surgical access assembly in the absence of a surgical instrument in the surgical access assembly, and an instrument seal seals around a surgical instrument that has been inserted through the surgical access assembly.

The breadth of surgical instrumentation on the market today requires a robust seal capable adjusting to multiple sizes and withstanding multiple insertions of surgical instrumentation. Some of the instrumentation can include sharp edges that can tear or otherwise damage seals. Therefore, it would be beneficial to have a surgical access assembly with improved seal durability.

### SUMMARY

A surgical access assembly including a valve assembly is provided. The surgical access assembly includes an instrument valve housing and the valve assembly. The instrument valve housing includes upper, lower, and inner housing sections and defines a cavity. The valve assembly is disposed within the cavity of the instrument valve housing. The valve assembly includes a guard assembly including first and second guard members, a seal assembly disposed adjacent to the guard assembly, and a centering mechanism for maintaining the seal assembly and guard assembly centered within the cavity of the instrument valve. Each of the first and second guard members defines a central axis and includes a plurality of slits extending radially outward from the central axis. The first guard member is oriented relative to the second guard member such that the plurality of slits of the first guard member is radially offset from the plurality of slits of the second guard member.

In embodiments, the first and second guard members each define three slits. The first and second guard members may be formed of nylon. The seal assembly may be formed of a polyisoprene or a silicone elastomer. The plurality of slits may form a star shape.

The surgical access assembly may further include a retainer assembly having upper and lower retainer members. At least one of the upper and lower retainer members includes a plurality of pins receivable through the guard assembly and seal assembly for retaining the guard and seal assemblies relative to each other. The centering mechanism may include a bellows.

In another aspect, a valve assembly including a guard assembly, a seal assembly disposed adjacent to the guard assembly, and a centering mechanism for maintaining the seal assembly and guard assembly centered within a cavity of an instrument valve is provided. The guard assembly includes first and second guard members. Each of the first and second guard members defines a central axis and includes a plurality of slits extending radially outward from the central axis. The first guard member is oriented relative to the second guard member such that the plurality of slits of the first guard member are radially offset from the plurality of slits of the second guard member.

A guard assembly for use in a valve assembly is also provided. The guard assembly includes a first guard member defining a central axis and including a plurality of slits extending radially outward from the central axis, and a second guard member defining a central axis and including a plurality of slits extending radially outward from the central axis. The first guard member is oriented relative to the second guard member such that the plurality of slits of the first guard member is radially offset from the plurality of slits of the second guard member.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure, wherein:
FIG. 1 is a side perspective view of a surgical access assembly according to an embodiment of the present disclosure;
FIG. 2 a side cross-sectional view of the surgical access assembly shown in FIG. 1 taken along section line 2-2;
FIG. 3 is an exploded perspective view of a valve assembly, with parts separated, including a centering mechanism, a guard assembly, a seal assembly, and a retainer assembly;
FIG. 4 is a perspective view of the seal assembly shown in FIG. 3;
FIG. 5 is a perspective view of a guard member of the guard assembly shown in FIG. 3;
FIG. 5A is a perspective view of a guard member of a guard assembly according to another embodiment of the present disclosure;
FIG. 6 is a top view of the seal assembly shown in FIG. 4; and
FIG. 7 is a cross-sectional side view of the seal assembly shown in FIG. 4 taken along section line 7-7.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals refer to similar or identical elements throughout the description of the figures.

As used herein, the term "distal" refers to that portion of the instrument, or component thereof which is farther from the user while the term "proximal" refers to that portion of the instrument or component thereof which is closer to the user.

Surgical access assemblies with obturators are employed during minimally invasive surgery, e.g., laparoscopic surgery, and provide for the sealed access of surgical instruments into an insufflated body cavity, such as the abdominal cavity. The surgical access assemblies of the present disclosure include an instrument valve housing mounted on a cannula tube, and include an obturator (not shown) inserted through the valve housing and cannula. The obturator can have a blunt distal end, or a bladed or non-bladed penetrating distal end and can be used to incise the abdominal wall so that the surgical access assembly can be introduced into the abdomen. The handle of the obturator can engage or selectively lock into the instrument valve housing of the surgical access assembly.

Surgical access assemblies are employed to tunnel through an anatomical structure, e.g., the abdominal wall, either by making a new passage through the anatomical structure or by passing through an existing opening through the anatomical structure. Once the surgical access assembly with the obturator has tunneled through the anatomical structure, the obturator is removed, leaving the surgical access assembly in place. The instrument valve housing of the surgical access assembly includes valves that prevent the escape of insufflation fluid from the body cavity, while also allowing surgical instruments to be inserted into the body cavity.

In various embodiments, a bladeless optical trocar obturator may be provided that permits separation of tissue planes in a surgical procedure and visualization of body tissue fibers as they are being separated, thereby permitting a controlled traversal across a body wall. In other embodiments, the trocar obturator may be bladeless without being optical, e.g., without providing contemporaneous visualization thereof through the distal tip of an obturator. The bladeless obturator may be provided for the blunt dissection of the abdominal lining during a surgical procedure.

Various trocar obturators suitable for use with the surgical access assembly of the present disclosure are known and include, for example, bladed, bladeless, blunt, optical, and non-optical. For a detailed description of the structure and function of exemplary trocar assemblies, including exemplar trocar obturators and exemplar cannulas, please refer to PCT Publication No. WO 2016/186905 ("the '905 publication"), the content of which is hereby incorporated by reference herein in its entirety.

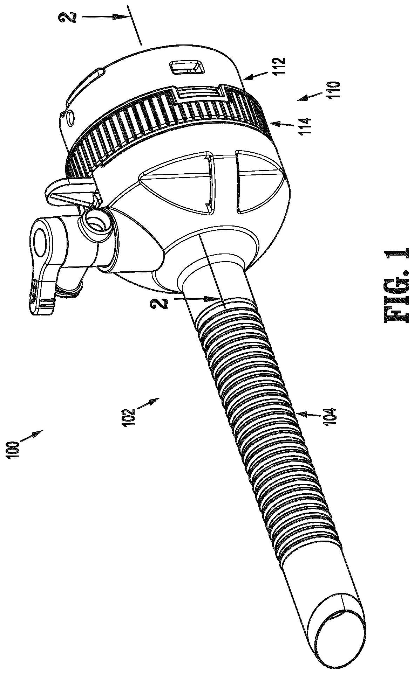

With initial reference now to FIG. 1, a surgical access assembly according to aspects of the present disclosure is shown generally as surgical access assembly 100. The surgical access assembly 100 includes a cannula 102 and an instrument valve housing 110 secured to the cannula 102. For a detailed description of an exemplary surgical access assembly, please refer to the '905 publication.

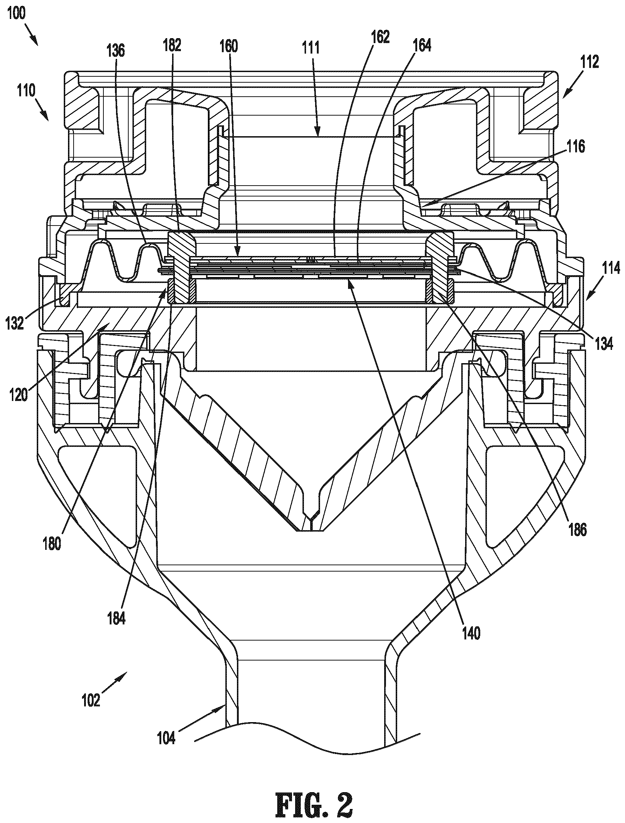

With reference to FIG. 2, the instrument valve housing 110 of the surgical access assembly 100 includes an upper housing section 112, a lower housing section 114, and an inner housing section 116. The upper, lower, and inner housing sections 112, 114, 116 are configured to support a valve assembly 120 on a proximal end of the cannula 102. More particularly, the inner housing section 116 is secured between the upper and lower housing sections 112, 114, and the valve assembly 120 is received between the inner and lower housing sections 116, 114. The upper and lower housing section 112, 114 of the instrument valve housing 110 may be selectively attachable to, and detachable from, the inner housing section 116. The lower housing section 114 may be releasably or permanently attached to a cannula tube 104 (FIG. 1) of the cannula 102. In embodiments, either or both of the upper and lower housing sections 112, 114 of the instrument valve housing 110 may include knurls, indentations, tabs, or be otherwise configured to facilitate engagement by a clinician.

The surgical access assembly 100 may also include features for the stabilization of the surgical access assembly. For example, the distal end of the cannula tube 104 may carry a balloon anchor or another expandable member that engages the abdomen from the interior side. For example, see U.S. Pat. No. 7,300,448, the entire disclosure of which is hereby incorporated by reference herein. A feature on the opposite side of the abdominal wall may be used to further stabilize the surgical access assembly, such as adhesive tabs or adjustable foam collars.

The upper, lower, and inner housing sections 112, 114, 116 of the instrument valve housing 110 define a longitudinal passage 111 for receipt of a surgical instrument (not shown). The valve assembly 120 is supported within the instrument valve housing 110 to provide sealed passage of the surgical instrument through the surgical access assembly 100.

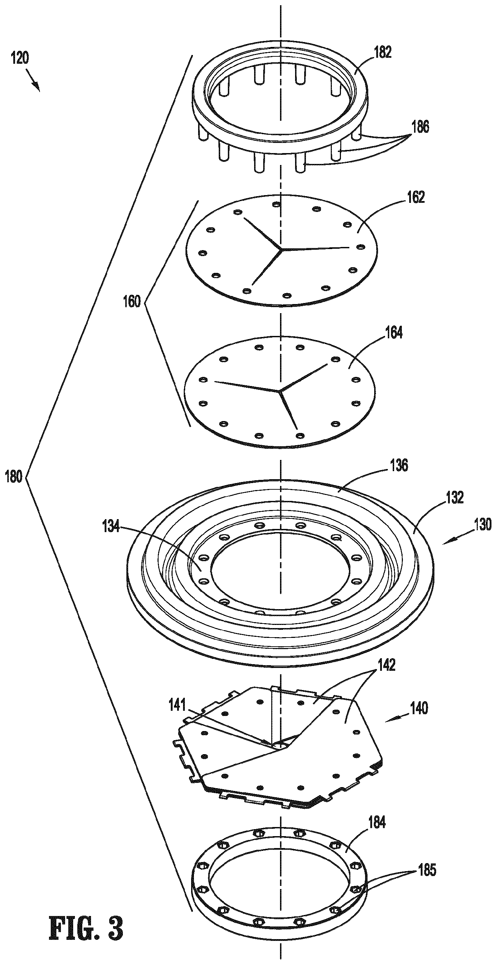

With particular reference to FIGS. 2 and 3, the valve assembly 120 supported in the instrument valve housing 110 (FIG. 2) includes a centering mechanism 130, a guard assembly 140, a seal assembly 160, and a retainer assembly 180. The centering mechanism 130 of the valve assembly 120 permits radial movement of the valve assembly 120 relative to the instrument valve housing 110 when a surgical instrument is received through the valve assembly 120, and returns the valve assembly 120 to a generally centered position once the surgical instrument is withdrawn from within the instrument valve housing 110. The guard assembly 140 protects the seal assembly 160 during insertion and withdrawal of a surgical instrument through the seal assembly 160. The seal assembly 160 provides sealed passage of the surgical instrument through the instrument valve housing 110. The retainer assembly 180 maintains the centering mechanism 130, the guard assembly 140, and the seal assembly 160 in an aligned relationship with one another.

With continued reference to FIGS. 2 and 3, as noted above, the centering mechanism 130 of the valve assembly 120 is configured to maintain the valve assembly 120 centered within the instrument valve housing 110 (FIG. 2). In embodiments, and as shown, the centering mechanism 130 includes an outer annular ring 132, an inner annular ring 134, and a bellows 136 disposed between the outer annular ring 132 and the inner annular ring 134. As shown in FIG. 2, the outer annular ring 132 is received between the inner housing section 116 and the lower housing section 114 to retain the centering mechanism 130 within the instrument valve housing 110. The inner annular ring 134 supports the seal assembly 160. For a detailed description of the structure and function of an exemplary centering mechanism, please refer to U.S. Pat. No. 6,702,787, the content of which is incorporated herein by reference in its entirety.

Although shown including the centering mechanism 130 having bellows 136, the valve assembly 120 may include alternative centering mechanisms. For example, the centering mechanism may include an annular base and a plurality of spokes extending from the base, as described in U.S. Pat. App. Pub. No. 2015/0025477 ("the '477 publication"), the content of which is incorporated herein by reference in its entirety. It is envisioned that the centering mechanism may include multiple sets of spokes, as disclosed in the '477 publication.

With continued reference to FIGS. 2 and 3, the seal assembly 140 of the valve assembly 120 is configured to provide a seal around an outer surface of a surgical instrument (not shown) passing through the instrument valve housing 110 (FIG. 2).

The seal assembly 140 includes a plurality of petals 142 defining an opening 141 therebetween to facilitate sealed passage of a surgical instrument (not shown) through the seal assembly 140. By forming the opening 141 out of the plurality of sections 142 instead of as a continuous solid opening through a single seal member, the likelihood of any of the petals of the plurality of petals 142 of the seal assembly 140 tearing during insertion, removal, and use of a surgical instrument therethrough is greatly reduced. Although shown including six (6) sections, it is envisioned that the seal assembly may include any suitable number of sections, and the sections may include flap portions of any size or configuration. For exemplary seal assemblies, as well as other aspects of surgical access assemblies, please refer to U.S. Patent Nos. 5,895,377 and 6,569,120 ("the '377 and '120 patents"), the entire disclosures of which are hereby incorporated by reference herein. For detailed description of the structure and function of other exemplary guard assemblies, please refer to commonly owned U.S. Pat. App. Ser. Nos. ##/###,####, 16/238,823, and ##/###,#### (Atty. Docket No. 203-12169, 203-12267, and 203-12319), the entire disclosures of which are incorporated herein by reference in its entirety.

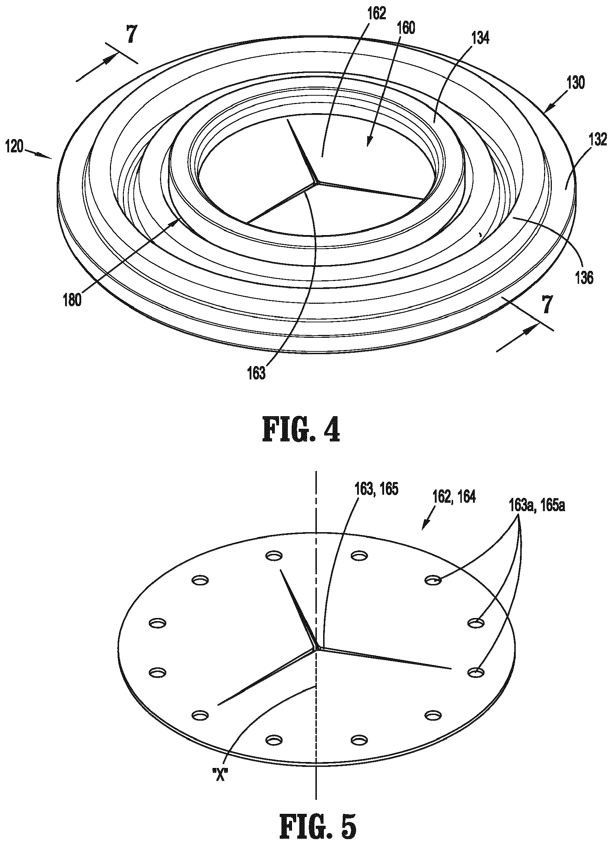

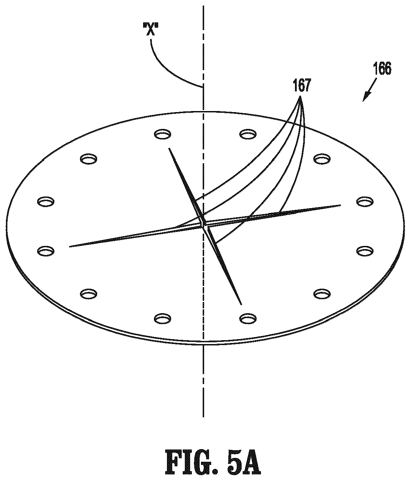

With additional reference to FIGS. 5-7, the guard assembly 160 of the valve assembly 120 includes a first guard member 162 and a second guard member 164. Although shown including only two guard members, it is envisioned that the guard assembly 160 may include more than two guard members. Each of the first and second guard members 162, 164 is a substantially planar disc. The first and second guard members 162, 164 may be formed of nylon or other suitable tear resistant material. The first and second guard members 162, 164 may be formed by stamping, molding or other suitable techniques.

Each of the first and second guard members 162, 164 of the guard assembly 160 defines a plurality of openings 163a, 165a about an outer circumference the respective first and second guard members 162, 164. The plurality of openings 163a, 165a are configured to receive a plurality of pins 186 of the retainer assembly 180 for retaining the guard assembly 160 relative to the centering mechanism 130 and the seal assembly 140.

Each of the first and second guard members 162, 164 of the guard assembly 160 defines a plurality of slits 163, 165, respectively. In embodiments, and as shown, the plurality of slits 163, 165 form a 3-pointed star radiating from a central axis "x" of the respective first and second guard members 162, 164. It is envisioned that the plurality of slits 163, 165 may form a star of any number of points, e.g., 4 (FIG. 5A) or 5, or may form a single slit extending across a portion of the diameter of the guard member. The greater the number of slits in the guard member, the lower the force required for insertion and retraction of a surgical instrument through the valve assembly 120.

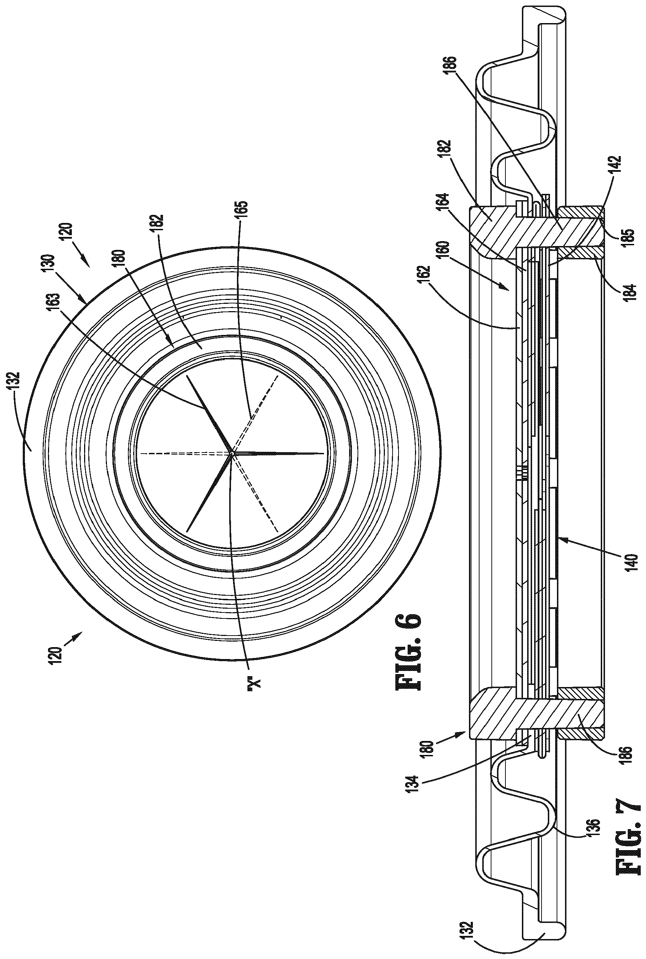

With particular reference to FIG. 6, the first and second guard members 162, 164 of the guard assembly 160 are oriented relative to each other such that the slits 163 of the first guard member 162 do not overlap the slits 165 of the second guard member 164. In this manner, when a surgical instrument passes through the valve assembly 120, the first and second guard members 162, 164 engage the entire outer circumference of the surgical instrument to protect the seal assembly 140 and prevent the seal assembly 140 from disintegrating into the body cavity during minimally invasive procedures.

Turning briefly to FIG. 5A, a guard member according to another embodiment of a guard assembly is shown generally as third guard member 166. The third guard member 166 is substantially similar to the first and second guard members 162, 164, and with therefore only be described as relates to the differences therebetween. The third guard member 166 defines a plurality of slits 167. The plurality of slits 167 form a 3-pointed star radiating from a central axis "x" of the third guard member 166.

In embodiments, the third guard member 166 combined with one or more additional guard members 166 to form a guard assembly (not shown). As discussed above with regards to the guard assembly 160, a first third guard members 166 of the alternative guard assembly (not shown) may be rotated relative to the adjacent third guard member 166 such that the plurality of slits 167 of the third guard members 166 are offset from one another. In embodiments, the third guard member 166 is rotated forty-five degrees (45°) relative to the adjacent third guard assembly 166.

It is envisioned that the guard assembly may include one or more of the first and second guard members 162, 164 and one or more third guard members 166. The guard members may be arranged with a third guard member 166 disposed between first and second guard members 162, 164. Alternatively, thirds guard member 166 may be disposed on either side of the first and second guard members 162, 164.

With reference to FIG. 7, the retainer assembly 180 (of the valve assembly 120 is configured to secure the guard assembly 140 relative to the seal assembly 160, and secure the guard and seal assemblies 140, 160 to the centering mechanism 130. The retainer assembly 180 includes an upper retainer member 182, and a lower retainer member 184.

As noted above, the upper retainer member 182 of the retainer assembly 180 includes a plurality of pins 186. The plurality of pins 186 extend from a bottom surface of the upper retainer member 182. Each pin 186 of the plurality of pins 186 is configured to be lockingly received within an opening 185 of a plurality of openings 185 (FIG. 3) of the lower retainer member 184. In embodiments, the plurality of pins 186 is welded, glued, adhered, bonded or otherwise secured within the plurality of openings 185 in the lower retainer member 184 to secure the upper retainer member 182 and the lower retainer member 184 together. Alternatively, the lower retainer member 184 may instead, or additionally, include a plurality of pins (not shown) with the upper retainer member 182 defining a plurality corresponding openings (not shown). Either or both of the upper and lower retainer members 182, 184 may include locking features (not shown) for engaging the plurality of pins and securing the upper retainer member 182 to the lower retainer member 184.

With continued reference to FIG. 7, the plurality of pins 186 of the upper retainer member 182 extend through the first and second guard members 162, 164 of the guard assembly 160, through the seal assembly 140, through the inner annular ring 134 of the centering mechanism 130, and into the plurality of openings 185 in the lower retainer member 184. As noted above, by placing the guard assembly 160 proximal of the seal assembly 140, thereby reducing or eliminating potential damage to the seal assembly 140 during insertion and retraction of a surgical instrument through the seal assembly 140. The placement of the guard assembly 160 also reduces the potential of the seal assembly 140 disintegrating into the body cavity during minimally invasive procedures.

During a surgical procedure utilizing surgical access assembly 100 (FIG. 1), a surgical instrument (not shown) is introduced into the instrument valve housing 110 through the longitudinal passage 111 in the upper, lower, and inner housing sections 112, 114, 116 (FIG. 2). As described in the '377 and '120 patents, the distal end of the surgical instrument engages the first and second guard members 162, 164 of the guard assembly 160 causing flaps of the respective first and second guard members 162, 164 to flex downward into contact with the seal assembly 140 to cause the central opening 141 of the seal assembly 140 to open to accommodate passage of the surgical instrument through the seal assembly 140. The guard assembly 160 minimizes damage to the seal assembly 140 during insertion of an instrument through the valve assembly 120. The guard assembly 160 operates to protect the seal assembly 140 from tearing or other damage as a surgical instrument is received through and withdrawn from the valve assembly 120.

While various embodiments of the present disclosure have been shown and described herein, it will be obvious to those skilled in the art that these embodiments are provided by way of example only. Numerous variations, changes, and substitutions will now occur to those skilled in the art without departing from the present disclosure. Accordingly, it is intended that the invention be limited only by the spirit and scope of the appended claims.

The invention may be described by reference to the following numbered paragraphs:-
1. A surgical access assembly comprising:
   an instrument valve housing including upper, lower, and inner housing sections and defining a cavity; and
   a valve assembly disposed within the cavity of the instrument valve housing, the valve assembly including:
      a guard assembly including first and second guard members, each of the first and second guard members defining a central axis and including a plurality of slits extending radially outward from the central axis, wherein the first guard member is oriented relative to the second guard member such that the plurality of slits of the first guard member are radially offset from the plurality of slits of the second guard member;
      a seal assembly disposed adjacent to the guard assembly; and
      a centering mechanism for maintaining the seal assembly and guard assembly centered within the cavity of the instrument valve.
2. The surgical access assembly of paragraph 1, wherein the first and second guard members each define three slits.
3. The surgical access assembly of paragraph 1, wherein the first and second guard members are formed of nylon.
4. The surgical access assembly of paragraph 1, wherein the seal assembly is formed of a polyisoprene or a silicone elastomer.
5. The surgical access assembly of paragraph 1, wherein the plurality of slits forms a star shape.
6. The surgical access assembly of paragraph 1, further including a retainer assembly including upper and lower retainer members.
7. The surgical access assembly of paragraph 6, wherein at least one of the upper or lower retainer members includes a plurality of pins receivable through the guard assembly and seal assembly for retaining the guard and seal assemblies relative to each other.
8. The surgical access assembly of paragraph 1, wherein the centering mechanism includes a bellows.
9. A valve assembly comprising:
   a guard assembly including first and second guard members, each of the first and second guard members defining a central axis and including a plurality of slits extending radially outward from the central axis, wherein the first guard member is oriented relative to the second guard member such that the plurality of slits of the first guard member is radially offset from the plurality of slits of the second guard member;
   a seal assembly disposed adjacent to the guard assembly; and
   a centering mechanism for maintaining the seal assembly and guard assembly centered within a cavity of an instrument valve.
10. The valve assembly of paragraph 9, wherein each of the first and second guard members has three slits.
11. The valve assembly of paragraph 9, wherein the first and second guard members are formed of nylon.
12. The valve assembly of paragraph 9, wherein the seal assembly is formed of a polyisoprene or a silicone elastomer.
13. The valve assembly of paragraph 9, wherein the plurality of slits form a star shape.
14. The valve assembly of paragraph 9, further including a retainer assembly including upper and lower retainer members.
15. The valve assembly of paragraph 14, wherein at least one of the upper or lower retainer members includes a plurality of pins receivable through the guard assembly and seal assembly for retaining the guard and seal assemblies relative to each other.
16. The valve assembly of paragraph 9, wherein the centering mechanism includes a bellows.
17. A guard assembly for use in a valve assembly, the guard assembly comprising:
   a first guard member defining a central axis and including a plurality of slits extending radially outward from the central axis; and
   a second guard member defining a central axis and including a plurality of slits extending radially outward from the central axis, wherein the first guard member is oriented relative to the second guard member such that the plurality of slits of the first guard member is radially offset from the plurality of slits of the second guard member.
18. The guard assembly of paragraph 17, wherein each of the first and second guard members has three slits.
19. The guard assembly of paragraph 17, wherein the first and second guard members are formed of nylon.
20. The guard assembly of paragraph 17, wherein the seal assembly is formed of a polyisoprene or a silicone elastomer.

## Claims

1. A surgical access assembly comprising:
an instrument valve housing including upper, lower, and inner housing sections and defining a cavity; and
a valve assembly disposed within the cavity of the instrument valve housing, the valve assembly including:
a guard assembly including first and second guard members, each of the first and second guard members defining a central axis and including a plurality of slits extending radially outward from the central axis, wherein the first guard member is oriented relative to the second guard member such that the plurality of slits of the first guard member are radially offset from the plurality of slits of the second guard member;
a seal assembly disposed adjacent to the guard assembly; and
a centering mechanism for maintaining the seal assembly and guard assembly centered within the cavity of the instrument valve.

2. The surgical access assembly of claim 1, wherein the first and second guard members each define three slits.

3. The surgical access assembly of claim 1 or claim 2, wherein the first and second guard members are formed of nylon.

4. The surgical access assembly of any preceding claim, wherein the seal assembly is formed of a polyisoprene or a silicone elastomer; and/or wherein the plurality of slits forms a star shape.

5. The surgical access assembly of any preceding claim, further including a retainer assembly including upper and lower retainer members; preferably wherein at least one of the upper or lower retainer members includes a plurality of pins receivable through the guard assembly and seal assembly for retaining the guard and seal assemblies relative to each other.

6. The surgical access assembly of any preceding claim, wherein the centering mechanism includes a bellows.

7. A valve assembly comprising:
a guard assembly including first and second guard members, each of the first and second guard members defining a central axis and including a plurality of slits extending radially outward from the central axis, wherein the first guard member is oriented relative to the second guard member such that the plurality of slits of the first guard member is radially offset from the plurality of slits of the second guard member;
a seal assembly disposed adjacent to the guard assembly; and
a centering mechanism for maintaining the seal assembly and guard assembly centered within a cavity of an instrument valve.

8. The valve assembly of claim 7, wherein each of the first and second guard members has three slits.

9. The valve assembly of claim 7 or claim 8, wherein the first and second guard members are formed of nylon; and/or wherein the seal assembly is formed of a polyisoprene or a silicone elastomer.

10. The valve assembly of any of claims 7 to 9, wherein the plurality of slits form a star shape; preferably further including a retainer assembly including upper and lower retainer members.

11. The valve assembly of claim 10, wherein at least one of the upper or lower retainer members includes a plurality of pins receivable through the guard assembly and seal assembly for retaining the guard and seal assemblies relative to each other.

12. The valve assembly of any of claims 7 to 11, wherein the centering mechanism includes a bellows.

13. A guard assembly for use in a valve assembly, the guard assembly comprising:
a first guard member defining a central axis and including a plurality of slits extending radially outward from the central axis; and
a second guard member defining a central axis and including a plurality of slits extending radially outward from the central axis, wherein the first guard member is oriented relative to the second guard member such that the plurality of slits of the first guard member is radially offset from the plurality of slits of the second guard member.

14. The guard assembly of claim 13, wherein each of the first and second guard members has three slits.

15. The guard assembly of claim 13 or claim 14, wherein the first and second guard members are formed of nylon; and/or wherein the seal assembly is formed of a polyisoprene or a silicone elastomer.
